(19) Europäisches Patentamt
European Patent Office
Office européen des brevets

(11) EP 1 543 142 B1

(12) **EUROPEAN PATENT SPECIFICATION**

(45) Date of publication and mention of the grant of the patent:
**26.11.2008 Bulletin 2008/48**

(21) Application number: **03755679.2**

(22) Date of filing: **22.09.2003**

(51) Int Cl.:
***C12N 1/20*** *(2006.01)* ***C12P 21/02*** *(2006.01)*
***C07K 7/56*** *(2006.01)* ***A01N 63/02*** *(2006.01)*
*C12R 1/125* *(2006.01)*

(86) International application number:
**PCT/JP2003/012087**

(87) International publication number:
**WO 2004/029273 (08.04.2004 Gazette 2004/15)**

(54) **PRODUCTION METHOD FOR ITURIN A AND ITS HOMOLOGUES**

HERSTELLUNGSVERFAHREN FÜR ITURIN A UND DESSEN HOMOLOGE

PROCEDE RELATIF A LA PRODUCTION D'ITURINE A ET D'HOMOLOGUES CORRESPONDANTS

(84) Designated Contracting States:
**AT BE BG CH CY CZ DE DK EE ES FI FR GB GR HU IE IT LI LU MC NL PT RO SE SI SK TR**

(30) Priority: **24.09.2002 JP 2002277873**
**27.09.2002 US 413755 P**

(43) Date of publication of application:
**22.06.2005 Bulletin 2005/25**

(73) Proprietor: **Showa Denko K.K.**
**Tokyo 105-8518 (JP)**

(72) Inventors:
- **Yoneda, Tadashi**
**Corp. R & D Center, Showa Denko KK**
**Chiba 267-0056 (JP)**
- **Kitakuni, Eiichi**
**Corp. R&D Center, Showa Denko K.K**
**Chiba-shi**
**Chiba 267-0056 (JP)**
- **Furuya, Kazuo**
**Corp. R&D Center, Showa Denko K.K.**
**Chiba-shi**
**Chiba 267-0056 (JP)**

(74) Representative: **Strehl Schübel-Hopf & Partner**
**Maximilianstrasse 54**
**80538 München (DE)**

(56) References cited:
**EP-A- 0 646 649** **WO-A-98/50422**
**WO-A-03/013251**

- **DATABASE CA [Online] CHEMICAL ABSTRACTS SERVICE, COLUMBUS, OHIO, US; TAKAZANE, SHIGERU ET AL: "Influences of Bacillus species in shoyu koji on shoyu brewing. 1. Isolation and identification of antifungal antibiotics producing bacteria" retrieved from STN Database accession no. 128:281993 XP002270870 -& NIPPON SHOYU KENKYUSHO ZASSHI (1998), 24(2), 77-82 , XP008027724**
- **DATABASE CA [Online] CHEMICAL ABSTRACTS SERVICE, COLUMBUS, OHIO, US; TAKAZANE, SHIGERU ET AL: "Influences of Bacillus species in shoyu koji on shoyu brewing. 2. Influences of antifungal Bacillus amyloliquefaciens on shoyu brewing" retrieved from STN Database accession no. 130:37495 XP002270871 -& NIPPON SHOYU KENKYUSHO ZASSHI (1998), 24(6), 341-346 , XP008027722**
- **HBID CHOUKRI ET AL: "Influence of the production of two lipopeptides, iturin A and surfactin S1, on oxygen transfer during Bacillus subtilis fermentation" APPLIED BIOCHEMISTRY AND BIOTECHNOLOGY, vol. 57-58, no. 0, 1996, pages 571-579, XP008027727 ISSN: 0273-2289 cited in the application**
- **SHODA M: "PRODUCTION OF MICROBIAL PESTICIDE IN SUBMERGED AND IN SOLID STATE FERMENTATIONS" MEDEDELINGEN - FACULTEIT LANDBOUWKUNDIGE EN TOEGEPASTE BIOLOGISCHE WETENSCHAPPEN, UNIVERSITEIT GENT, GENT,, BE, vol. 64, no. 5A, 1999, pages 275-280, XP001126255 ISSN: 1373-7503**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 543 142 B1

- **OHNO AKIHIRO ET AL: "Production of the antifungal peptide antibiotic, iturin by Bacillus subtilis NB22 in solid state fermentation" JOURNAL OF FERMENTATION AND BIOENGINEERING, vol. 75, no. 1, 1993, pages 23-27, XP008027721 ISSN: 0922-338X**
- **PHAE C-G ET AL: "INVESTIGATION OF OPTIMAL CONDITIONS FOR FOAM SEPARATION OF ITURIN AN ANTIFUNGAL PEPTIDE PRODUCED BY BACILLUS-SUBTILIS" JOURNAL OF FERMENTATION AND BIOENGINEERING, vol. 71, no. 2, 1991, pages 118-121, XP008027720 ISSN: 0922-338X cited in the application**
- **PATENT ABSTRACTS OF JAPAN vol. 009, no. 078 (C-274), 6 April 1985 (1985-04-06) -& JP 59 212416 A (AJINOMOTO KK;OTHERS: 01), 1 December 1984 (1984-12-01) cited in the application**

## Description

TECHNICAL FIELD

The present invention relates to a method of producing iturin A and its homologues by cultivating a microbe belonging to *Bacillus* in a liquid medium containing soybean or its extract as a nitrogen source to accumulate iturin A and its homologues at high concentrations in the liquid medium. Also, the present invention relates to a culture product containing iturin A and its homologues, to a solid substance containing iturin A and its homologues obtained by drying the culture product, and to application methods thereof.

BACKGROUND ART

It has been conventionally known that microbes belonging to the genus *Bacillus,* particularly *Bacillus subtilis,* produce iturin A and its homologues (cf., for example, Besson et al., Journal of Antibiotics, 1978, Vol. 31, p.284-288).
Iturin A and its homologues refer to a group of compounds having a structure represented by formula 1 shown below and have antimicrobial activities on fungi, particularly plant pathogenic microbes, so that they have attracted attention as excellent components preventing plant disease.

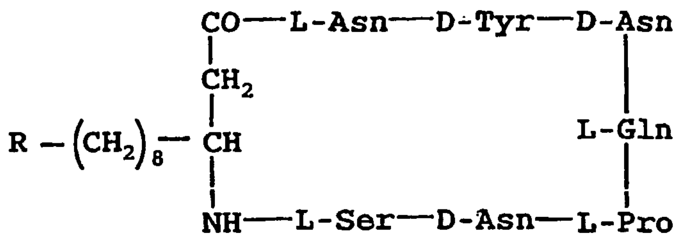

(1)

(R represents a linear or branched alkyl group having 3 to 10 carbon atoms.)
Examples of methods for producing iturin A and its homologues include those described in JP-A-59-212416, JP-A-7-143897 (U.S. Patent Nos.5,470,827 and 5,494,809)and so forth.
However, the *Bacillus* microbes produce iturin A and its homologues at productivities too low to apply to industrial-scale production. Accordingly, many researchers have made efforts to increase productivities at which iturin A and its homologues are produced.
Hatada et al. disclose a method for producing iturin A-based substance by aerobic culture of *Bacillus subtilis* No. NA-apb-1 strain in a nutrient medium (JP-B-63-20519). In the publication, there are taught as nitrogen sources, peptone, meat extract, yeast extract, casein hydrolysates, corn starch liquor, gluten meal, and inorganic nitrogen sources and it is described that 270 mg of iturin A is obtained from 30 L of a culture filtrate obtained by cultivating the strain in a medium containing peptone, meat extract and yeast extract. Sandrin et al. disclose that 480 mg/L iturin A and its homologues can be obtained in a synthetic medium containing proline as a nitrogen source (Biotechnology and Applied Biochemistry, 1990, Vol. 12, p.370-375).
Hbid et al. disclose a method that achieves a yield of iturin A and its homologues of 1, 388 mg/L in a medium containing peptone (Applied Biochemistry and Biotechnology, 1996, Vol. 57/58, p.571-579). Phae et al. disclose a method that achieves a yield of iturin A and its homologues of 620 mg/L (Journal of Fermentation and Bioengineering, 1991, Vol. 71, p.118-121).
On the other hand, as a method for cultivating a *Bacillus* microbe in a liquid medium containing soybean powder or its extract, the inventors of the present invention disclose a production method for surfactin (JP-A-2002-176993). However, the publication discloses nothing about iturin A and its homologues. Further, JP-A-59-212416 discloses performing cultivation in a medium containing 1% soybean powder to obtain 300 mg of iturin A and its homologues from 16 L of the culture broth. However, the amount of iturin A obtained is little more than the amount obtained by a known cultivation method.
It is unknown that cultivation of a *Bacillus* microbe having the ability of producing iturin A and its homologues in a liquid medium containing soybean powder or its extract in an amount of 2 mass% or more enables accumulation of iturin A and its homologues in the medium in a concentration of 1.5 g/L or more.
WO 9850422 discloses a method for producing iturin A compounds comprising cultivating a Bacillus subtilis microbe.

DISCLOSURE OF THE INVENTION

The productivity of iturin A and its homologues conventionally obtained is insufficient for industrial applications and a production method that gives an increased productivity has been demanded.

Accordingly, an object of the present invention is to provide a method for producing iturin A and its homologues by cultivating a *Bacillus* microbe that produces iturin A and its homologues and allows the microbe to accumulate iturin A and its homologues in the culture broth in high concentrations.

Another object of the present invention is to provide a culture product containing iturin A and its homologues, solid product thereof and a method for using them.

With a view to achieving the above-mentioned objects, the inventors of the present invention have made extensive studies on various components in culture media. As a result, they have found that cultivation of a *Bacillus* microbe that produces iturin A and its homologues in a medium containing 2 mass% or more of soybean pulverisates or its extract as a nitrogen source results in accumulation of iturin A and its homologues in the culture broth in high concentrations, thereby accomplishing the present invention.

That is, the present invention relates to methods for producing iturin A and its homologues, to cultures containing iturin A and its homologues, and to solids containing iturin A and its analogues described below.

1. A method for producing iturin A and its homologues, comprising cultivating a *Bacillus* microbe having an ability to produce iturin A and its homologues in a liquid medium containing 2 mass% or more of soybean powder or its extract to allow the microbe to accumulate iturin A and its homologues in the medium in a concentration of 1.5 g/ℓ or more, wherein the *Bacillus* microbe having an ability to produce iturin A and its homologues is a *Bacillus* microbe that has no ability to accumulate surfactin in an amount of more than 50 ppm when cultivated in a medium containing soybean pulverisate or its extract as a nitrogen source, and wherein the *Bacillus* microbe having an ability to produce iturin A and its homologues is a *Bacillus* microbe that can grow in a medium containing 1.5 g/ℓ or more of iturin A and its homologues.
2. The method for producing iturin A and its homologues according to 1 above, wherein 0 to 3 mass% of phosphates, in terms of $K_2HPO_4$, is added to the liquid medium.
3. The method for producing iturin A and its homologues according to 1 or 2 above, wherein the microbe is *Bacillus subtilis.*
4. The method for producing iturin A and its homologues according to any one of 1 to 3 above, wherein the microbe is *Bacillus subtilis* having the accession number FFERM BP-08427.
5. The method for producing iturin A and its homologues according to 1 above, wherein the liquid medium containing 2 mass% or more of soybean powder or its extract contains at least one member selected from the group consisting of maltose, starch syrup, soluble starch, dextrin, glucose, sucrose, and fructose.
6. A Bacillus microbe having an ability to produce iturin A and its homologues and having no ability to accumulate surfactin in an amount of more than 50 ppm when cultivated in a medium containing soybean pulverisate or its extract as a nitrogen source, wherein the Bacillus microbe having an ability to produce iturin A and its homologues is a Bacillus microbe that can grow in a medium containing 1.5 g/l or more of iturin A and its homologues.
7. The Bacillus microbe according to 6 above which is Bacillus subtilis having the accession number FERM BP-08427.
8. A culture containing iturin A and its homologues and the Bacillus microbe according to claim 6 or 7 above.
9. A solid containing iturin A and its homologue obtainable by drying the culture according to 8 above.
10. An agent for preventing plant diseases, comprising the culture containing iturin A and its homologues or solid thereof according to 8 or 9 above.
11. A method for preventing a plant disease, comprising using the culture containing iturin A and its homologues or solid thereof according to 8 or 9 above in an unpurified form.

DETAILED DESCRIPTION OF THE INVENTION

Hereinafter, the present invention will be described in detail.

In the present invention, iturin A and its homologues means derivatives represented by the following formula (1), including related compounds.

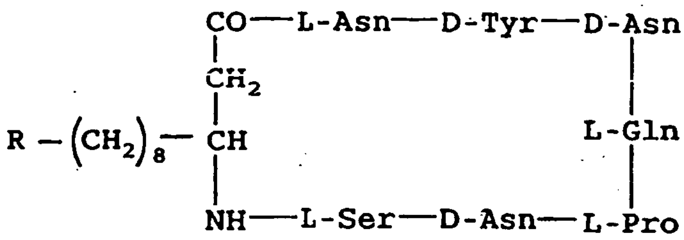

(1)

(R represents a linear or branched alkyl group having 3 to 10 carbon atoms).

According to the present invention, performing cultivation of a microbe that produces iturin A and its homologues in a medium by adding soybean powder or its extract as a nitrogen source in an amount of 2 mass% or more to the medium enables highly concentrated accumulation of iturin A and its analogues in the medium. So far as the present inventors know, the technique where a *Bacillus* microbe is cultivated in a medium containing soybean pulverisate or its extract as a nitrogen source has conventionally been known. However, the present inventors are first to find out the novel technique where a microbe that produces iturin A and its homologues is cultivated in a medium containing 2 mass% or more of soybean pulverisate or its extract as a nitrogen source to accumulate iturin A and its homologues in the medium in high concentrations.

The Bacillus microbe used in the present invention is not particularly limited so far as it produces iturin A and its homologues. Since iturin A and its homologues accumulate in the medium in high concentrations, it is necessary that the microbe can grow in the presence of a high concentration of iturin. Therefore, the Bacillus microbe is one that has an ability to produce iturin A and its homologues and can grow in a medium containing 1.5 g/ℓ of iturin A and its homologues. Further, as another preferable example, the Bacillus microbe is a Bacillus microbe that has an ability to produce iturin A and its homologues but has no ability to produce surfactin. "Having no ability to produce surfactin" referred to herein means that when a microbe is cultivated in a medium containing soybean pulverisate or its extract as a nitrogen source, accumulation amount of surfactin is 50 ppm or less. Preferable examples of Bacillus microbe that has an ability to produce iturin A and its homologues and can grow in a medium containing 1.5 g/ℓ or more of iturin and its homologue, include *Bacillus subtilis* having the accession number FERM BP-08427.

*Bacillus subtilis* having the accession number FERM BP-08427, isolated from compost, has the following mycological characteristics. Bacteriological Properties

(a) Morphology

(1) Form of the bacterium: rod,
(2) Size of the bacterium:

0.7 to 0.9 × 1.5 to 3.0 µm,

(3) Polymorphism: no
(4) motility: yes
(5) spore: present,
form of spore: elliptical or cylindrical
(6) Gram stain: positive
(7) Acid resistance: negative

(b) Growth conditions on meat juice agar plate culture:

circular colony with a diameter of 1 to 2 mm, having an undulate periphery, viscous, no lust;

(c) Physiological properties

(1) Reduction of nitrates: positive
(2) VP test: positive
(3) Production of indole: negative
(4) Utilization of citric acid: positive

(5) Utilization of succinic acid: negative
(6) Utilization of propionic acid: negative
(7) Utilization of tartaric acid: negative
(8) Urease: negative
(9) Oxidase: positive
(10) Catalase: positive
(11) Growth range: pH 5 to 9, temperature 20 to 50°C
(12) 10% NaCl Medium: growth
(13) Anaerobic culture: negative
(14) Egg yolk reaction: negative
(15) Hydrolysis of starch: positive
(16) Decomposition of arginine: positive
(17) Decomposition of tyrosine: negative
(18) Liquefaction of gelatin: positive
(19) Decomposition of aesculin: positive
(20) OF test: oxidative
(21) Acid production from glucose: negative

*Bacillus subtilis* having the accession number FERM BP-08427 was deposited at National Institute of Advanced Industrial Science and Technology, at AIST Tsukuba Central 6, 1-1, Higashi 1-Chome, Tsukuba-shi, Ibaraki-ken, Japan (postal code: 305-8566)(Accession No. FERM P-19032) and was transferred from the original deposit to international deposit on July 10, 2003(No. FERM BP-08427).

Further, in the present invention, mutants obtained by spontaneous mutation of the deposited *Bacillus subtilis* strain can also be used preferably. Mutants may be obtained as spontaneous mutant strains by selecting those having an altered colony morphology on a plate medium or by reacting a chemical, or physical mutation inducing factor with the deposited *Bacillus subtilis* strain to produce a stock of bacterial strains with altered yields of iturin A and its homologues and therefrom isolating a colony that has an increased productivity.

As a chemical mutation inducing factor, for example, EMS (ethyl methanesulfonate), diethyl sulfate, or NTG (N-methyl-N'-nitro-N-nitrosoguanidine) can be used. As a physical mutation inducing factor, ultraviolet rays, gamma rays, X-rays or so forth can be used in an amount required to induce a mutation.

An example of method for producing a stock of mutants is a method where *Bacillus subtilis* cells grown up to a logarithmic stage in a nutrient medium such as NB (Nutrient Broth; manufactured by Difco Laboratories, Inc.) are collected and suspended in physiological saline after washing, a mutation inducing amount of NTG is added to the cells to induce mutation, and then again the cells are collected, washed to remove NTG and cultivated in a nutrient medium such as NB (Nutrient Broth; manufactured by Difco Laboratories, Inc.) to produce a stock of mutant strains.

Examples of method for isolating a colony having an increased productivity include a method where an appropriately diluted stock of mutants is spread to grow *Bacillus subtilis* cells on a plate medium prepared by adding agar to a medium such as TBAB (Tryptose Blood Agar Base; manufactured by Difco Laboratories, Inc.) with sheep blood added therein, and a colony having a larger clear zone around the colony than other colonies is isolated to select mutant strains that can produce iturin A and its homologues in high concentrations.

Subsequently, iturin A and its homologues productivity of the mutant of *Bacillus* subtilis thus isolated can be confirmed by cultivating the deposited *Bacillus subtilis* strain as a control in a test tube.

Hereinafter, the method for producing iturin A and its homologues according to the present invention will be described. The method for producing iturin A and its homologues according to the present invention can most conveniently be performed, for example, as follows. The deposited *Bacillus subtilis* strain is cultivated in a nutrient medium such as an L medium at 25 to 42°C, preferably 28 to 38°C, for about 5 to about 24 hours, and the obtained culture broth in an amount of 0.1 to 10 mass%, preferably 0.5 to 7 mass%, more preferably 1 to 5 mass%, is inoculated in a medium containing soybean powder or its extract as a nitrogen source. This is cultivated at a temperature of 25 to 42°C, preferably 28 to 35°C, for about 30 to about 150 hours. In the case where the temperature is outside the above-mentioned temperature range, production of iturin A and its homologues is considerably decreased undesirably.

In the present invention, "soybean powder or its extract" means grainy soybean powder obtained by pulverizing soybean or defatted soybean, pulverized soybean powder obtained by pulverizing soybean into fine powder, extracts of them (for example, hot water extracts), hydrolysates (for example, acid hydrolysates, enzyme hydrolysates) and so forth. The concentration of the soybean powder or its extract is desirably 2 mass% or more. However, on the other hand, since the soybean powder or its extract in too high a concentration might cause insufficient sterilization, it is desirable that the concentration of the soybean powder or its extract should not exceed 20 mass%. Accordingly, the concentration of the soybean powder or its extract for obtaining a high productivity is 2 to 20 mass%, preferably 3 to 17 mass%, more preferably 4 to 14 mass%.

The medium used in the present invention may contain besides the soybean powder or its extract, usually used catabolizable carbon sources, nitrogen sources and inorganic salts and so forth. Further, amino acids and/or vitamins and so forth may be added, if necessary.

Examples of the catabolizable carbon sources include glucose, maltose, sucrose, fructose, soluble starch, starch syrup, dextrin, molasses, potato extract, malt, peat, beet, plant oil, corn steep liquor, fructose, syrup, sugar, liquid sugar, invert sugar, alcohols, organic acids, organic acid salts, alkanes or other general carbon sources. These can be used singly or in combination. Among them, maltose, soluble starch, starch syrup, and dextrin are preferred. These can be used in concentrations of usually about 0.01 to about 50 mass%, preferably about 1 to about 40 mass%.

Further, as the catabolizable nitrogen sources, those containing inorganic or organic nitrogen, for example, ammonium salts such as ammonium nitrate, ammonium sulfate, ammonium chloride, ammonium acetate, ammonium carbonate and ammonium bicarbonate, ammonia, sodium nitrate, potassium nitrate, sodium glutamate, urea, peptone, meat extract, corn steep liquor, casein hydrolysates, feather meal, and yeast extract can be utilized. These can be used singly or in combination. They can be advantageously used in concentrations of usually about 0.01 to 30 mass%, preferably 0.1 to 10 mass%.

Further, as the inorganic components, cations or anions, such as potassium ion, sodium ion, magnesium ion, phosphate ion, iron ion, manganese ion, calcium ion, zinc ion, cobalt ion, nickel ion, copper ion, molybdenum ion, sulfate ion, chloride ion, or nitrate ion can be added. The amount of the inorganic component to be added may vary depending on cultivation conditions. Usually, the magnesium salt is added to a concentration of about 10 ppm to about 2 mass%, and in case of salts other than phosphate, the salt is added to a concentration of about 0.1 ppm to about 1,000 ppm. Phosphate ions may be added as phosphate salts. If the phosphate salt is added to a concentration higher than 3 mass% in terms of $K_2HPO_4$, the concentration of accumulated iturin A and its homologue is decreased. Therefore, it is desirable that the phosphate salt be added to a concentration of 3 mass% or less. More preferably, a medium without addition of phosphate ion is preferable.

Examples of amino acids to be added include L-glycine, L-alanine, L-valine, L-leucine, L-isoleucine, L-serine, L-threonine, L-phenylalanine, L-tyrosine, L-cysteine, cystine, L-methionine, L-tryptophane, L-histidine, L-proline, L-aspartic acid, L-asparagine, L-glutamic acid, L-glutamine, L-arginine, L-lysine, D-valine, D-isoleucine, and so forth. One or more of these may be added. The amino acid is added to a concentration of about 0.001 to about 5 mass%, preferably about 0.01 to about 1 mass%.

As the vitamin, one or more of biotin, thiamine, riboflavin, pyridoxine, nicotinic acid, nicotinamide, pantothenic acid, pyridoxal, myo-inositol, choline, folic acid, cobalamine, cyanocobalamine, and so forth may be added. The vitamin is added to a concentration of 0.1 to 100 ppm, preferably 1 to 50 ppm.

In the cultivation according to the present invention, the above-mentioned medium is placed in a vessel such as a test tube, a flask, or a fermentation tank and cultivation is performed with vigorous aeration.

In the case of cultivation using a vessel such as a test tube or a flask, aeration is performed by vigorously shaking, and initial pH of the medium is adjusted to 6.5 to 8.0. In the case where high concentration production is performed by using a vessel such as a fermentation tank, cultivation is performed under a sterile air flow while agitating. In the case where foaming occurs to such an extent that cultivation is difficult, a common antifoaming agent usually used may be added.

The pH of the medium is maintained at 6.0 to 9.0, preferably 6.5 to 8.0, more preferably 6.8 to 7.3. The adjustment of pH is performed by addition of a basic aqueous solution such as an aqueous ammonia solution, an aqueous potassium hydroxide solution, an aqueous sodium carbonate solution or an aqueous potassium carbonate solution. Among them, it is preferable to use an aqueous ammonia solution. The concentration of the aqueous ammonia solution advantageously is about 8 to about 25 mass%. By performing such cultivation under preferable conditions, a culture containing iturin A and its homologues that contains iturin A and its homologues in a concentration of 1.5 g/L or more can be obtained in 30 to 150 hours.

By drying the above-mentioned culture by a known method such at freeze-drying or spray drying, a solid containing iturin A and its homologues can be obtained. The culture containing iturin A and its homologues or the solid containing iturin A and its homologues, which exhibits an effect of preventing plant diseases when applied to the soil of farmland or crop leaves, is useful. The culture containing iturin A and its homologues and the solid containing iturin A and its homologues are also embraced by the present invention.

Further, iturin A and its homologues can be recovered from the culture containing iturin A and its homologues and purified. Purification can be performed by a known method, for example, a method in which a culture is made acidic by addition of sulfuric acid, hydrochloric acid, nitric acid or the like to precipitate iturin A and its homologues, and then the precipitates are subjected to extraction treatment with an organic solvent such as methanol, ethanol, or chloroform, treatment with activated carbon, crystallization treatment and/or the like.

The iturin A and its homologues obtained according to the present invention can be used not only as agents for preventing plant diseases but also used as detergents, emulsifiers, humidifiers, dispersants, solubilizers, antistatic agents, antifogging agents, lubricants and so forth. The iturin A and its homologue obtained according to the present

invention are useful as compounding ingredient for cosmetics, foods, pharmaceuticals, agricultural chemicals and so forth.

BEST MODE FOR CARRYING OUT THE INVENTION

Hereinafter, the present invention will be described in more detail by examples. However, the present invention should not be considered to be limited to the examples.

Preparation Example 1 for obtaining a mutant of the deposited *Bacillus subtilis* strain.

The deposited *Bacillus subtilis* strain was inoculated in 5 ml of an L medium (10 g of peptone, 5 g of yeast extract, 5 g of sodium chloride, and water to make 1 liter) and incubated at 35°C at 300 rpm for 16 hours. Then, the obtained culture was inoculated in 5 ml of the same medium at 1 v/v% and incubated at 35°C at 300 rpm until OD660 reached 0.2. Thereafter, the cells were recovered by centrifugation. The supernatant was discarded. The recovered cells were washed with 5 ml of PBS buffer (0.8 w/v% NaCl, 0.02 w/v% KCl, 0.144 w/v% $Na_2HPO_4$, and 0.024 w/v% $KH_2PO_4$, adjusted to pH 7.4 with HCl) three times and suspended again in 0.5 ml of the same buffer.

0.05 ml of an aqueous solution of 2,000 ppm N-methyl-N'-nitro-N-nitrosoguanidine was added to the suspension and the mixture was left to stand at 30°C for 10 minutes. The suspension was centrifuged, the supernatant was discarded, and the cells was washed with 5 ml of the same buffer three times and suspended again in 1 ml of a fresh L medium. The suspension was added to 4 ml of an L medium, and subjected to cell growth at 35°C for one night. Thereafter, 2.5 ml of an aqueous 50 mass% glycerol solution was added to the suspension, and aliquots thereof were dispensed into vials for cryopreservation and frozen at -135°C to be preserved as transformants.

Then, the preserved transformants diluted with sterilized water were plated at a concentration of 50, 000/plate on agar plate media each containing 5 w/v% sheep blood, 4 w/v% of glucose, and 0.1 w/v% NB (manufactured by Difco Laboratories, Inc.) and 0.1 w/v% yeast extract (Applied Environmental Microbiology, 42:408-412 (1981)) so that approximately 200 colonies/plate were obtained. After incubation at 35°C for 20 to 48 hours, clear zones formed around the grown colonies were observed and 50,000 colonies that formed big clear zones were selected.

The deposited *Bacillus subtilis* strain and the obtained 50, 000 colonies were streaked on L plate media and grown at 35°C for one night. 1 ml aliquots of a medium having the following composition A were dispensed into test tubes to each of which one loopful of the L plate medium was inoculated and incubated at 35°C for 72 hours.

| <Composition A> | (Mass%) |
|---|---|
| Soybean powder | 8 |
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |
| Ion-exchanged water | the balance |

The culture was centrifuged and iturin A and its homologues contained in the supernatant were determined by a HPLC method under the following conditions.

Sample size: 10 μl
Column: Shodex Silica C18P4E, 4. 6 mm x 250 mm, manufactured by Showa Denko K. K.
Column temperature: 40°C
Eluant: Acetonitrile : 10 mM ammonium acetate aqueous solution = 35:75 (v/v)
Flow rate: 1.5 ml/min
Detector: UV detector
Wavelength: 205 nm

The determination was performed by preparing a calibration curve using standard samples of iturin A and its homologues (manufactured by Sigma-Aldrich Co.).

A mutant strain (Mutant 1) that produces iturin A and its homologues with high productivity, showing an increased concentration of accumulated iturin A and its homologues as compared with the original strains of the deposited *Bacillus subtilis* strain, was obtained.

Preparation Example 2 for obtaining a mutant of the deposited *Bacillus subtilis* strain.

The concentration of surfactin in the supernatant of the culture in Preparation Example 1 was determined by a HPLC method under the following conditions.

Sample size: 20 μl
Column: Shodex Silica C18P4E, 4. 6 mm x 250 mm, manufactured by Showa Denko K. K.
Column temperature: 40°C
Eluant: Acetonitrile : 19 mM trifluoroacetic acid aqueous solution = 80:20 (v/v)
Flow rate: 1.0 ml/min
Detector: UV detector
Wavelength: 205 nm

The determination was performed by preparing a calibration curve using a standard sample of surfactin (manufactured by Sigma-Aldrich Co.).
A mutant strain (Mutant 2) that produces substantially no surfactin, with concentration of accumulated surfactin being 50 ppm or less, was obtained.

Example 1: Effect of a nitrogen source on production of iturin A and its homologues in test tube cultivation

Strains of the deposited *Bacillus subtilis* strain were streaked on L plate media and grown at 35°C for one night. 1 ml aliquots of a medium having the following composition B were dispensed into test tubes to each of which one loopful of the L plate medium was inoculated and incubated at 35°C for 72 hours.

| <Composition B> | (Mass%) |
|---|---|
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |
| Nitrogen source* | 2.0 |
| Ion exchange water | the balance |

*The nitrogen source is one selected from the group consisting of soybean powder, potassium nitrate, ammonium nitrate, ammonium sulfate, urea, sodium glutamate and peptone.

The culture was centrifuged and the concentrations of iturin A and its homologues contained in the supernatant were determined by a HPLC method.
The concentration of accumulated iturin A and its homologues in each case using the nitrogen source was as follows.

| nitrogen source used | concentration of iturin A and its homologues |
|---|---|
| Soybean powder | 1.5 g/L |
| Potassium nitrate | 0.03 g/L |
| Ammonium nitrate | 0.03 g/L |
| Ammonium sulfate | 0.03 g/L |
| Urea | 0.03 g/L |
| Sodium glutamate | 0.1 g/L |
| Peptone | 0.15 g/L |

Example 2: Effect of soybean powder concentration on production of iturin A and its, homologues in test tube cultivation

Strains of the deposited *Bacillus subtilis* strain were streaked on L plate media and grown at 35°C for one night. 1 ml aliquots of media having the following compositions C, D, and E, respectively, were dispensed into test tubes to each of which one loopful of the L plate medium was inoculated and incubated at 35°C for 72 hours.

| <Composition C> | (Mass%) |
|---|---|
| Soybean powder | 1 |
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |
| Ion exchange water | the balance |

| <Composition D> | (Mass%) |
|---|---|
| Soybean powder | 2 |
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |
| Ion exchange water | the balance |

| <Composition E> | (Mass%) |
|---|---|
| Soybean powder | 8 |
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |
| Ion exchange water | the balance |

The culture was centrifuged and the concentration of iturin A and its homologues contained in the supernatant were determined by a HPLC method.

The concentration of accumulated iturin A and its homologues in each case using the medium was as follows.

| medium used | concentration of iturin A and its homologues |
|---|---|
| Composition C: | 0.3 g/L |
| Composition D: | 1.5 g/L |
| Composition E: | 3.8 g/L |

Example 3: Effect of a carbon source on production of iturin A and its homologues in test tube cultivation

Strains of the deposited *Bacillus subtilis* strain were streaked on L plate media and grown at 35°C for one night. 1 ml aliquots of a medium having the following composition F to which the following carbon source was added were dispensed into test tubes to each of which one loopful of the L plate medium was inoculated and incubated at 35°C for 72 hours.

| <Composition F> | (Mass%) |
|---|---|
| Soybean powder | 8 |
| $K_2HPO_4$ | 0.5 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Carbon source** | 6.7 |
| Ion exchange water | the balance |

**The carbon source is one selected from the group consisting of maltose, soluble starch, starch syrup, dextrin, glucose, sucrose and fructose.

The culture was centrifuged and the accumulation concentrations of iturin A and its homologues contained in the supernatant were determined by a HPLC method. The accumulation concentrations of iturin A and its homologues in each case using the carbon source was as follows.

| | |
|---|---|
| Maltose | 3.8 g/L |
| Soluble starch | 3.8 g/L |
| Starch syrup | 3.8 g/L |
| Dextrin | 3.8 g/L |
| Glucose | 2.8 g/L |
| Sucrose | 2.2 g/L |
| Fructose | 2.3 g/L |

Example 4: Effect of a phosphate on production of iturin A and its homologues in test tube cultivation

Strains of the deposited *Bacillus subtilis* strain were streaked on L plate media and grown at 35°C for one night. 1 ml aliquots of a medium each having the following composition G to which $K_2HPO_4$ having a concentration (1) to (6) was added were dispensed into test tubes to each of which one loopful of the L plate medium was inoculated and incubated at 35°C for 72 hours.

| <Composition G> | (Mass%) |
|---|---|
| Soybean powder | 8 |
| $MgSO_4 \cdot 7H_2O$ | 0.05 |
| $FeSO_4 \cdot 7H_2O$ | 0.0025 |
| $MnSO_4 \cdot 5H_2O$ | 0.0022 |
| $CaCl_2$ | 0.0184 |
| Maltose | 6.7 |

| Concentration of $K_2HPO_4$ | |
|---|---|
| (1) | 0 mass% (no addition) |
| (2) | 0.1 mass% |
| (3) | 0.5 mass% |
| (4) | 1.5 mass% |
| (5) | 3.0 mass% |
| (6) | 4.5 mass% |
| Ion exchange water | the balance |

After adjustment to pH 7 with sodium carbonate, the culture was centrifuged and the accumulation concentrations of iturin A and its homologues contained in the supernatant were determined by a HPLC method. The accumulation concentration of iturin A and its homologues in each case where $K_2HPO_4$ having each concentration was added to the medium was as follows.

| Concentration of $K_2HPO_4$ | Concentration of iturin A and homologues |
|---|---|
| (1) 0 mass% | 3.8 g/L |
| (2) 0.1 mass% | 3.6 g/L |
| (3) 0.5 mass% | 3.5 g/L |
| (4) 1.5 mass% | 3.0 g/L |
| (5) 3.0 mass% | 2.2 g/L |
| (6) 4.5 mass% | 1.5 g/L |

Example 5: Production of iturin A and its homologues in a fermentation tank

The deposited *Bacillus subtilis* strain, mutant 1 and mutant 2 were streaked on L plate media and grown at 35°C for one night. One loopful each media was inoculated in a flask with a baffle to which 50 ml of an L medium was added and incubated at 35°C at 150 rpm for 8 hours. A medium having the following composition H was prepared in a 5-L fermentation tank and each culture of L plate medium was added thereto. While adjusting pH to 6.5 to 7.5 with 20% ammonia water, incubation was performed at 35°C for 150 hours.

| <Composition H> | |
|---|---|
| Soybean powder | 160 g |
| $MgSO_4 \cdot 7H_2O$ | 5 g |
| $FeSO_4 \cdot 7H_2O$ | 0.25 g |
| $MnSO_4 \cdot 5H_2O$ | 0.22 g |
| $CaCl_2$ | 1.84 g |
| Starch syrup | 450 g |
| Ion exchange water | 1,383 g |

The culture was centrifuged and iturin A and its homologues contained in the supernatant were determined by a HPLC method. The amounts of iturin A and its homologues were as follows.

| | |
|---|---|
| The deposited *Bacillus subtilis* strain | 3.8 g/L |
| Mutant 1 | 6.7 g/L |
| Mutant 2 | 6.7 g/L |

From the results, it is apparent that each of these strains can grow in the presence of 1.5 g/L or more of iturin A.

INDUSTRIAL APPLICABILITY

According to the present invention, iturin A and its homologues that are useful in various industrial fields such as pharmaceuticals, agricultural chemicals, foods, cosmetics, and chemicals can be produced by using inexpensive medium raw materials, with drastically increased concentrations as compared with the conventional methods.

Further, according to the present invention which enables production of iturin A and its homologues in a high concentration, in the field of agricultural chemicals and plant disease prevention, the culture as is can be used in applications where conventionally a culture itself cannot be used as it is due to insufficient concentration.

## Claims

1. A method for producing iturin A and its homologues, comprising cultivating a Bacillus microbe having an ability to produce iturin A and its homologues in a liquid medium containing 2 mass% or more of soybean powder or its extract to allow the microbe to accumulate iturin A and its homologues in the medium in a concentration of 1.5 g/l or more, wherein the Bacillus microbe having an ability to produce iturin A and its homologues is a Bacillus microbe that has

no ability to accumulate surfactin in an amount of more than 50 ppm when cultivated in a medium containing soybean pulverisate or its extract as a nitrogen source, and wherein the Bacillus microbe having an ability to produce iturin A and its homologues is a Bacillus microbe that can grow in a medium containing 1.5 g/l or more of iturin A and its homologues.

2. The method for producing iturin A and its homologues as claimed in claim 1, wherein 0 to 3 mass% of phosphates, in terms of $K_2HPO_4$, is added to the liquid medium.

3. The method for producing iturin A and its homologues as claimed in claim 1 or 2, wherein the microbe is Bacillus subtilis.

4. The method for producing iturin A and its homologues as claimed in any one of claims 1 to 3, wherein the microbe is Bacillus subtilis having the accession number FERM BP-08427.

5. The method for producing iturin A and its homologues as claimed in claim 1, wherein the liquid medium containing 2 mass% or more of soybean powder or its extract contains at least one member selected from the group consisting of maltose, starch syrup, soluble starch, dextrin, glucose, sucrose, and fructose.

6. A Bacillus microbe having an ability to produce iturin A and its homologues and having no ability to accumulate surfactin in an amount of more than 50 ppm when cultivated in a medium containing soybean pulverisate or its extract as a nitrogen source, wherein the Bacillus microbe having an ability to produce iturin A and its homologues is a Bacillus microbe that can grow in a medium containing 1.5 g/l or more of iturin A and its homologues.

7. The Bacillus microbe of claim 6 which is Bacillus subtilis having the accession number FERM BP-08427.

8. A culture containing iturin A and its homologues and the Bacillus microbe according to claim 6 or 7.

9. A solid containing iturin A and its homologues obtainable by drying the culture as claimed in claim 8.

10. An agent for preventing plant diseases, comprising the culture containing iturin A and its homologues or solid thereof as claimed in claim 8 or 9.

11. A method for preventing a plant disease, comprising using the culture containing iturin A and its homologues or solid thereof as claimed in claim 8 or 9 in an unpurified form.

**Patentansprüche**

1. Verfahren zum Herstellen von Iturin A und dessen Homologen, umfassend das Kultivieren eines Bacillus-Mikroorganismus mit der Fähigkeit zur Produktion von Iturin A und dessen Homologen in einem flüssigen Medium, das 2 Massen-% oder mehr Sojabohnenpulver oder dessen Extrakt enthält, wobei zugelassen wird, dass der Mikroorganismus Iturin A und dessen Homologe in dem Medium in einer Konzentration von 1,5 g/l oder mehr anhäuft, wobei der Bacillus-Mikroorganismus mit der Fähigkeit zur Produktion von Iturin A oder dessen Homologen ein Bacillus-Mikroorganismus ist, der Surfactin in einer Menge von mehr als 50 ppm, wenn er in einem Medium kultiviert wird, das Sojabohnenpulverisat oder dessen Extrakt als Stickstoffquelle enthält, nicht anhäufen kann, und wobei der Bacillus-Mikroorganismus mit der Fähigkeit zur Produktion von Iturin A und dessen Homologen ein Bacillus-Mikroorganismus ist, der in einem Medium wachsen kann, das 1,5 g/l oder mehr Iturin A und dessen Homologe enthält.

2. Verfahren zum Produzieren von Iturin A und dessen Homologen nach Anspruch 1, wobei 0 bis 3 Massen-% Phosphate, ausgedrückt als $K_2HPO_4$, zu dem flüssigen Medium gegeben werden.

3. Verfahren zum Produzieren von Iturin A und dessen Homologen nach Anspruch 1 oder 2, wobei der Mikroorganismus Bacillus subtilis ist.

4. Verfahren zum Produzieren von Iturin A und dessen Homologen nach einem der Ansprüche 1 bis 3, wobei der Mikroorganismus Bacillus subtilis mit der Hinterlegungsnummer FERM BP-08427 ist.

5. Verfahren zum Produzieren von Iturin A und dessen Homologen nach Anspruch 1, wobei das flüssige Medium, das

2 Massen-% oder mehr Sojabohnenpulver oder dessen Extrakt enthält, mindestens ein Mitglied enthält, das aus der aus Maltose, Stärkesirup, löslicher Stärke, Dextrin, Glucose, Saccharose und Fructose bestehenden Gruppe ausgewählt ist.

6. Bacillus-Mikroorganismus mit der Fähigkeit zur Produktion von Iturin A oder dessen Homologen, der Surfactin in einer Menge von mehr als 50 ppm, wenn er in einem Medium kultiviert wird, das Sojabohnenpulverisat oder dessen Extrakt als Stickstoffquelle enthält, nicht anhäufen kann, wobei der Bacillus-Mikroorganismus mit der Fähigkeit zur Produktion von Iturin A und dessen Homologen ein Bacillus-Mikroorganismus ist, der in einem Medium wachsen kann, das 1,5 g/l oder mehr Iturin A und dessen Homologe enthält.

7. Bacillus-Mikroorganismus nach Anspruch 6, der Bacillus subtilis mit der Hinterlegungsnummer FERM BP-08427 ist.

8. Iturin A und dessen Homologe und den Bacillus-Mikroorganismus nach Anspruch 6 oder 7 enthaltende Kultur.

9. Feststoff, der Iturin A und dessen Homologe enthält, erhältlich durch Trocknen der Kultur nach Anspruch 8.

10. Mittel zum Verhindern von Pflanzenkrankheiten, welches die Kultur enthält, die Iturin A und dessen Homologe oder den Feststoff davon nach Anspruch 8 oder 9 enthält.

11. Verfahren zum Verhindern von Pflanzenkrankheiten, welches die Verwendung der Kultur, die Iturin A und dessen Homologe oder den Feststoff davon nach Anspruch 8 oder 9 enthält, in ungereinigter Form umfasst.

**Revendications**

1. Procédé de production d'iturine A et de ses homologues comprenant la culture d'un microbe Bacillus ayant la capacité de produire de l'iturine A et ses homologues dans un milieu liquide contenant 2 % en masse ou plus de poudre de soja ou son extrait pour permettre au microbe d'accumuler de l'iturine A et ses homologues dans le milieu en une concentration 1,5 g/l ou plus, où le microbe Bacillus ayant la capacité de produire de l'iturine A et ses homologues est un microbe Bacillus qui n'a pas la capacité d'accumuler de la surfactine en une quantité supérieure à 50 ppm lorsqu'il est cultivé dans un milieu contenant du pulvérisat de soja ou son extrait comme source d'azote, et où le microbe Bacillus ayant la capacité de produire de l'iturine A et ses homologues est un microbe Bacillus qui peut se développer dans un milieu contenant 1,5 g/l ou plus d'iturine A et ses homologues.

2. Procédé de production d'iturine A et de ses homologues selon la revendication 1, où 0 à 3 % en masse de phosphates en termes de $K_2HPO_4$ sont ajoutés au milieu liquide.

3. Procédé de production d'iturine A et de ses homologues selon la revendication 1 ou 2, où le microbe est Bacillus subtilis.

4. Procédé de production d'iturine A et de ses homologues selon l'une quelconque des revendications 1 à 3, où le microbe est Bacillus subtilis ayant le numéro d'entrée FERM BP-08427.

5. Procédé de production d'iturine A et de ses homologues selon la revendication 1, où le milieu liquide contenant 2 % en masse ou plus de poudre de soja ou son extrait contient au moins un élément sélectionné dans le groupe constitué du maltose, du sirop d'amidon, de l'amidon soluble, du dextrose, du glucose, du saccharose et du fructose.

6. Microbe Bacillus ayant la capacité de produire de l'iturine A et ses homologues et n'ayant pas la capacité d'accumuler de la surfactine en une quantité supérieure à 50 ppm lorsqu'il est cultivé dans un milieu contenant du pulvérisat de soja ou son extrait comme source d'azote, où le microbe Bacillus ayant la capacité de produire de l'iturine A et ses homologues est un microbe Bacillus qui peut se développer dans un milieu contenant 1,5 g/l ou plus d'iturine A et ses homologues.

7. Microbe Bacillus selon la revendication 6, qui est Bacillus subtilis ayant le numéro d'entrée FERM BP-08427.

8. Culture contenant de l'iturine A et ses homologues et le microbe Bacillus selon la revendication 6 ou 7.

9. Solide contenant de l'iturine A et ses homologues susceptible d'être obtenu par séchage de la culture selon la

revendication 8.

10. Agent de prévention des maladies des végétaux, comprenant la culture contenant de l'iturine A et ses homologues ou le solide correspondant selon la revendication 8 ou 9

11. Procédé de prévention d'une maladie des végétaux comprenant l'utilisation de la culture contenant de l'iturine A et ses homologues ou le solide correspondant selon la revendication 8 ou 9 sous une forme non purifiée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 59212416 A
- JP 7143897 A
- US 5470827 A
- US 5494809 A
- JP 63020519 B
- JP 2002176993 A
- WO 9850422 A


### Non-patent literature cited in the description


- **BESSON et al.** *Journal of Antibiotics,* 1978, vol. 31, 284-288
- *Biotechnology and Applied Biochemistry,* 1990, vol. 12, 370-375
- *Applied Biochemistry and Biotechnology,* 1996, vol. 57 (58), 571-579
- **JOURNAL OF FERMENTATION AND BIOENGINEERING.** *Journal of Fermentation and Bioengineering,* 1991, vol. 71, 118-121
- *Applied Environmental Microbiology,* 1981, vol. 42, 408-412